# EUROPEAN PATENT SPECIFICATION

(11) **EP 1 968 446 B1**
(45) Date of publication and mention of the grant of the patent: **30.07.2014**
(21) Application number: 06844448.8
(22) Date of filing: 20.11.2006
(51) Int. Cl.: A61B 5/107

(54) **CERVICAL DILATION MEASUREMENT APPARATUS**
ZERVIKALES DILATATIONSMESSGERÄT
APPAREIL DE MESURE DE LA DILATATION DU COL DE L'UTERUS

(30) Priority: 29.12.2005 US 321061
(43) Date of publication of application: 17.09.2008
(73) Proprietor: Intrapartum, LLC, Jacksonville, FL 32256 (US)
(72) Inventor: DUBEY, Dharmesh K., Jacksonville, FL 32256 (US); BAIRD, Tim, Ponte Vedra Beach, FL 32082 (US)
(74) Representative: Zeitler - Volpert - Kandlbinder
(86) International application number: PCT/US2006/044961
(87) International publication number: WO 2007/078449

(56) References cited:
- WO-A-98/09565
- US-A- 4 141 345
- US-A- 4 245 656
- US-A- 4 611 603
- US-A- 6 066 104

## Description

The present invention relates to obstetric devices and more particularly, to a method and apparatus for measuring cervical dilation during pregnancy.

### BACKGROUND OF THE INVENTION

During the later stages of pregnancy, the cervix typically undergoes numerous physical changes which provide increased safety and ease with which the fetus can be delivered. Particularly, the cervical canal tissue softens and increases in pliability, and subsequently, the diameter of the cervical canal begins to increase. Eventually, the dilation of the cervix is completed, allowing for the unobstructed passage of the fetus.

Cervical diameter is monitored throughout labor and is instrumental in diagnosing such conditions as dysfunctional or arrested labor, to determine whether labor augmentation or a cesarean section should be performed, as well as to establish whether or when various pharmaceutical agents should be administered. Physical examination of the cervical diameter is generally performed by inserting two fingers into the vagina and up to the cervix. Upon reaching the cervix, the fingers are spread apart to determine the approximate dilated diameter. While an obstetrician may be fairly experienced in performing a manual cervical diameter measurement, the accuracy of such a measurement can be highly subjective and can further vary depending on the particular experience, judgment, and even finger size of the attending physician. Considering the importance of the cervical dilation measurement in assessing labor progression, it is crucial to provide dilation information that is precise as well as reproducible among different healthcare providers or physicians.

Given the subjectivity and probability of inaccurate or imprecise dilation measurements, it would be desirable to provide for the precise and accurate attainment of cervical dilation measurements on a repeat basis during the course of labor. US4611603, US6066104, US4245656, WO98/09565 and US 4141345 each disclose devices for monitoring cervical dilatation.

### SUMMARY OF THE INVENTION

The present invention advantageously provides a device for the accurate and precise measuring of cervical dilation during labor.

The present invention provides a cervical dilation sensor to aid in the manual, two-finger approach commonly employed.

The invention is defined by the subject-matter of the appended claim 1.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 is an illustration of an embodiment of a medical device ;
FIG. 2 is a side view of a distal end of the medical device of FIG. 1;
FIG. 3 is a cross-sectional view of a distal end of the medical device of FIG. 1;
FIG. 4 is an additional cross-sectional view of the medical device of FIG. 1;
FIG. 5 is a cross-sectional view of an embodiment of a dilation indicator ;
FIG. 6 is an illustration of a distal end of a medical device in a deflated state; ;
FIG. 7 is an illustration of a distal end of a medical device in an inflated state;
FIG. 8 is a perspective illustration of an embodiment of a cervical dilation sensor in accordance with the present invention;
FIG. 9 is a side view of the cervical dilation sensor of FIG. 8;
FIG. 10 is an additional illustration of the cervical dilation sensor of FIG. 8;
FIG. 11 is yet another depiction of the cervical dilation sensor of FIG. 8;
FIG. 12 shows an embodiment of a cervical dilation sensor coupled to a hand;
FIG. 13 depicts ari embodiment of a cervical dilation sensor within a glove;
FIG. 14 illustrates an additional embodiment of a cervical dilation sensor coupled to a hand; and
FIG. 15 shows an embodiment of a calibration element for use with a cervical dilation sensor in accordance with the present invention.

### DETAILED DESCRIPTION OF THE INVENTION

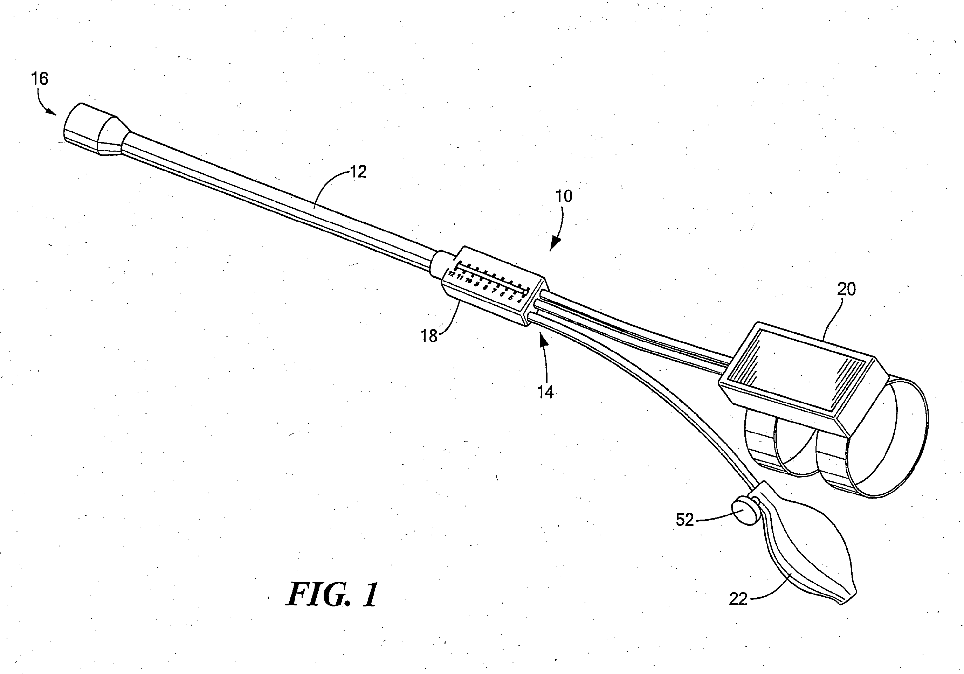

As shown in FIG. 1, an example provides a medical device 10 for measuring cervical dilation. The medical device 10 includes an elongate body 12 defining a proximal end 14 and a distal end 16. The medical device 10 may further include a dilation indicator 18 coupled to the proximal end 14 of the elongate body 12 that is capable of providing a visual indicator of the dilation measurement made by the medical device 10, as well as a control element 20 and an inflation source 22, which will be discussed in more detail below.

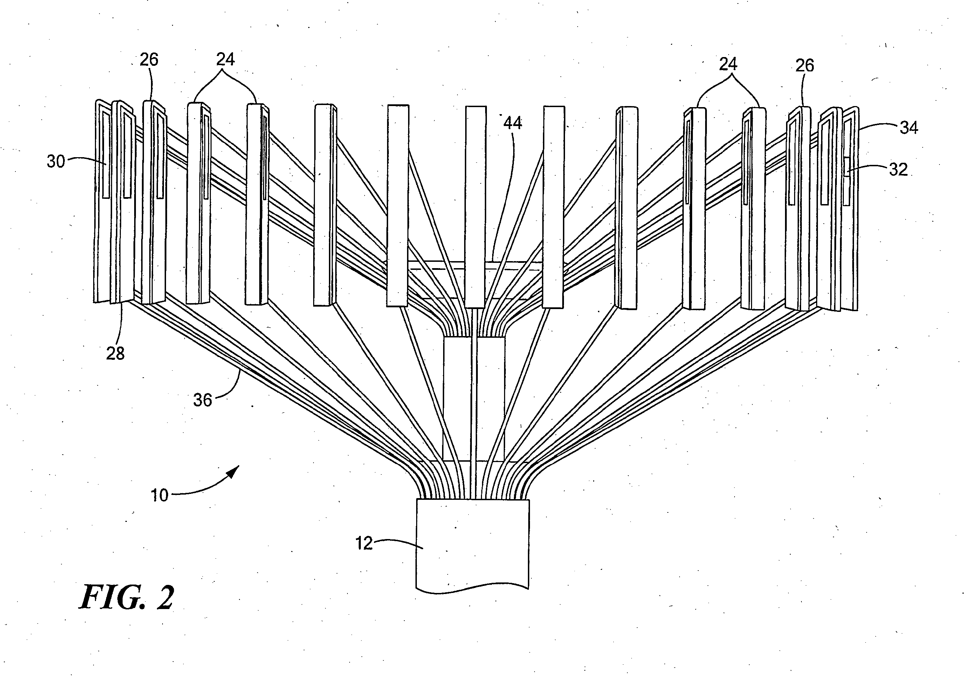

Now referring to FIG. 2, the medical device 10 may further include an array of movable elements 24 disposed circumferentially about an axis of the elongate body 12, where the array of movable elements 24 is located in proximity to the distal end 16 of the elongate body 12. The array of movable elements 24 are movable in a radial direction as to expand and contact with the tissue of the cervix when positioned for measurement of cervical dilation. Moreover, the array of movable elements 24 may be retracted upon completion of the desired measurement to ease the withdrawal of the medical device 10 from the patient. Each movable element may define an upper portion 26 and a lower portion 28. In addition, each movable element may define a channel 30 such that one or more pressure sensors 32 may be mounted or otherwise positionable within the channel 30 of the movable element Moreover, an outer cushion 34 may be coupled to an outer surface of each movable element, where the outer cushion 34 may be constructed from a gel-like material or other suitable padding. The array of movable elements 24 may further be inovably coupled to the elongate body 12 of the medical device 10 by a plurality of wires 36 coupled to the upper and lower portion 28s of the movable elements 24, where the plurality of wires 36 further extend through a length of the elongate body 12.

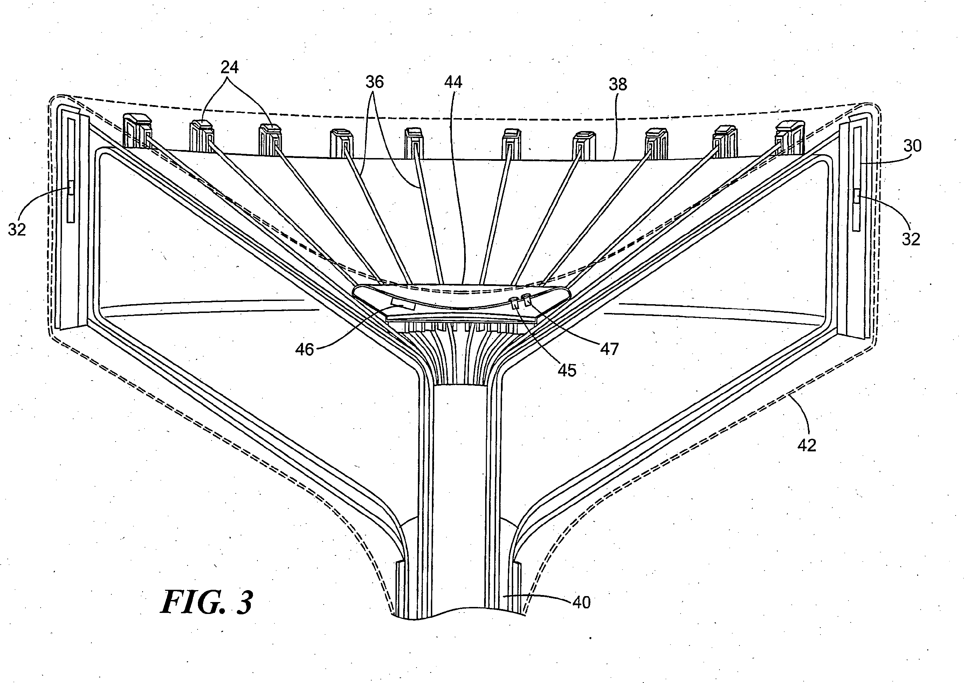

While the array of movable elements 24 may be extended and retracted by manipulating the plurality of wires 36, an actuating mechanism may be provided to facilitate movement of the array of movable elements 24 from a retracted position to an extended position, and vice versa. The actuating mechanism may include a spring mechanism, a telescoping element, or, alternatively, the medical device 10 may include an expandable element 38, such as a balloon. Now referring to FIG. 3, the medical device 10 may further include the expandable element 38 coupled to or otherwise disposed on the elongate body 12 at or near the distal end 16 of the elongate body 12. The expandable element 38 may be configured in a myriad of shapes, including a toroidal configuration in which the expandable element 38 defines a ring-like, "O" shape. Moreover, an inflation lumen 40 can be included in fluid communication with the expandable element 38, where the inflation lumen 40 is disposed within and traverses a substantial length of the elongate body 12.

The medical device 10 may include additional features providing safety, ease of use, and the like. For example, the medical device 10 may include a protective sheath 42 encasing at least a portion of the distal end 16 of the elongate body 12. The sheath 42 may include one or more layers of various materials to provide a water-tight seal around the medical device, as well as adding to patient comfort by having additional padding and / or a lubricious coating to ease positioning of the device. Furthermore, a distal pad 44 may be coupled to the elongate body 12 at or near the distal end 16, where the distal pad 44 may be contoured or shaped to conform to the curvature of the head of a baby. In addition, a distal pressure sensor 46 may be coupled to the distal pad 44 to aid in monitoring the positioning of the medical device 10 and for determining contact with the baby. The distal pad 44 and distal pressure sensor 46 may provide feedback to a physician and aid in the axial positioning of the medical device 10 upon insertion into a patient. Furthermore, a camera 45 and a lighting element 47 may also be coupled to the distal portion of the medical device. The camera 45 may be a miniaturized instrument or pin-hole camera as commonly employed in endoscopic surgical procedures, while the lighting element 47 may include a diode, fiber optic, or other illumination mechanism as is known in the art. The camera 45 and lighting element 47 may provide visual feedback to a physician to further aid in maneuvering and positioning the medical device when in use.

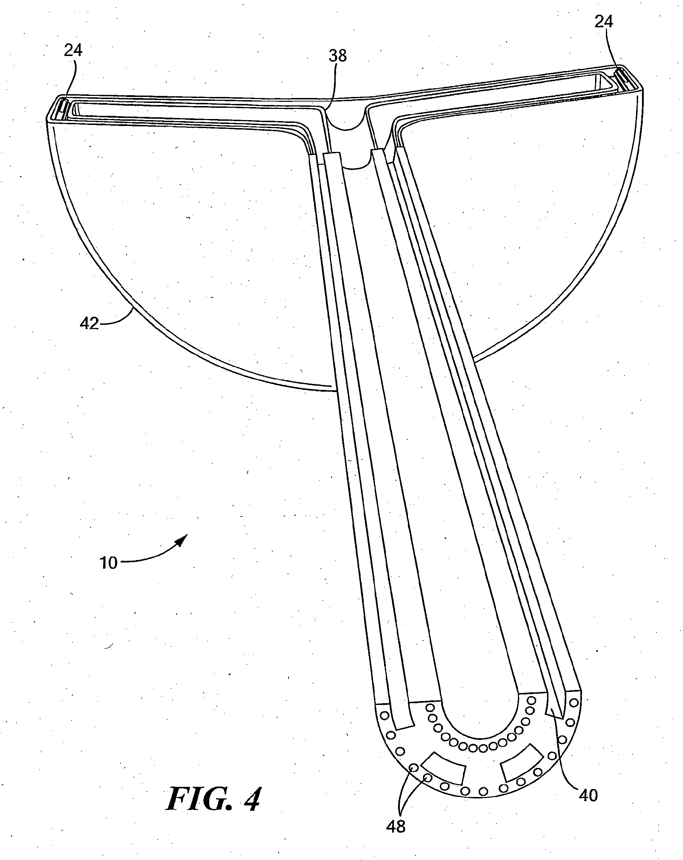

As shown in FIG. 4, the elongate body 12 may define a plurality of wire lumens 48 for slideably receiving a portion of each of the plurality of wires 36 coupled to the array of movable elements 24. Each wire of the plurality of wires 36 may be slideably positioned within each of the plurality of wire lumens 48 as to slide freely with little friction, thereby facilitating the movement of the array of movable elements 24 when the medical device 10 is in use. The wires 36 may have sufficient length as to extend through the entire length of the respective wire lumens 48, and may further extend out of the proximal end 14 of the elongate body 12.

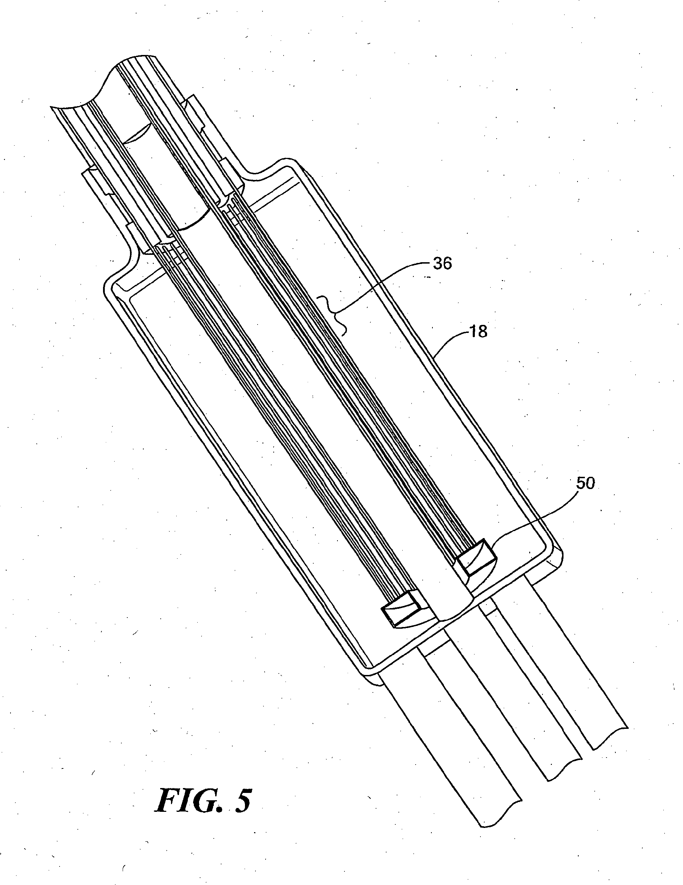

The medical device 10 may further include a measurement mechanism for monitoring and / or quantifying the movement of the array of movable elements 24 when the medical device 10 is in use. For example, as shown in the FIG. 5 illustration of a cross-section of the dilation indicator 18, the medical device 10 may include a tension ring 50 coupled to the plurality of wires 36 such that the tension ring 50 moves as the wires 36 extend and retract in response to the movement of the array of movable elements 24. The tension ring 50 may further be slideably coupled to the dilation indicator 18, where the dilation indicator 18 conveys a dilation measurement in response to the relative motion of the tension ring 50, the plurality of wires 36, and thus, the array of movable elements 24. The dilation indicator 18 may include predetermined values calculated from the movement of the tension ring 50 as to eliminate the need for a physician to do any calculating to determine the dilation measurement.

Again referring to FIG. 1, in an exemplary system, the proximal end 14 of the medical device 10 is coupled to the control element 20 which may be in communication with the numerous sensors provided on the medical device 10, and may also include a visual display to indicate the various operating characteristics and feedback from the device and the included sensors. The control element 20 may include an external console or may further include a wrist-mounted device to ease the overall use of the medical device 10, and may also be in communication with the camera 45 and lighting element 47 coupled to the distal end of the medical device 10. In addition, the inflation source 22 can be provided which may be coupled to the inflation lumen 40 at the proximal end 14 of the elongate body 12, where the inflation source 22 is able to provide a fluid or gas into the inflation lumen 40 for subsequent delivery to the expandable element 38. Examples of suitable inflation source 22s include manual pumps, powered pumps, or the like. Moreover, an exhaust valve 52 may be in fluid communication with both the inflation source 22 as well as the inflation lumen 40 for subsequent control of the release of fluid from the medical device 10.

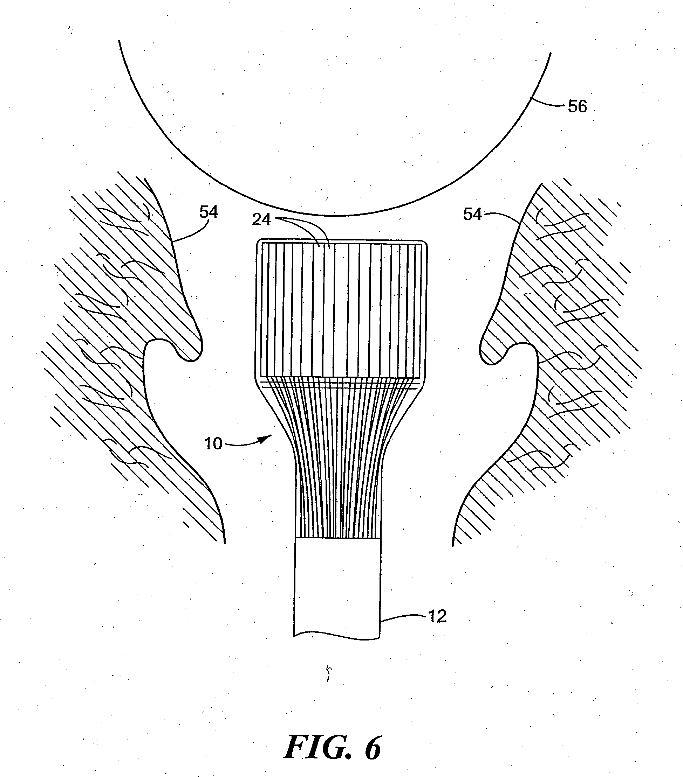

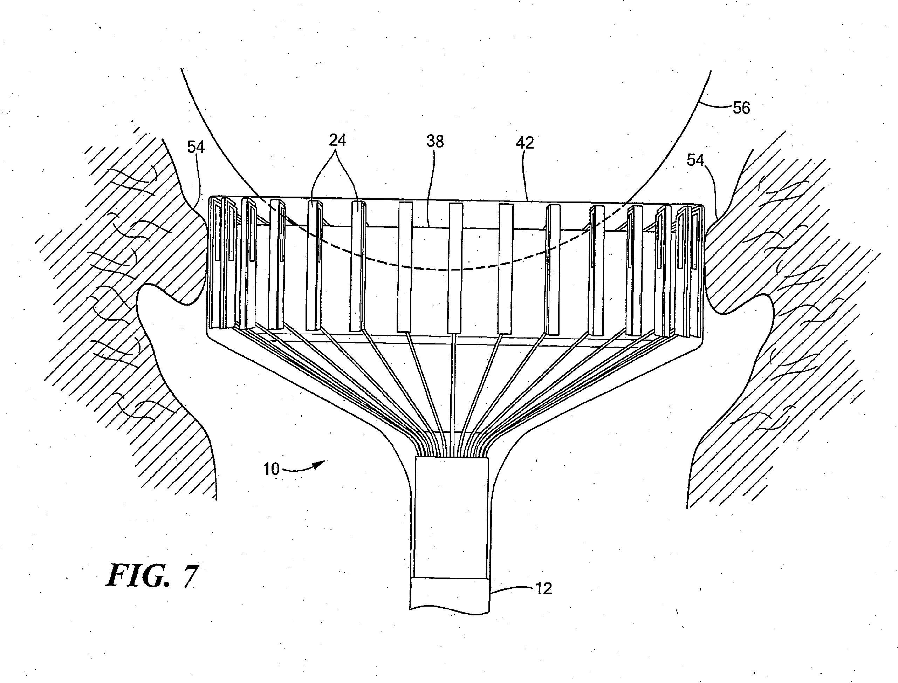

Referring now to FIGS. 6 and 7, in an exemplary use of the medical device 10 , a precise dilation measurement may be performed during the various stages of labor. The medical device 10, in a deflated state, may be positioned such that the distal end 16 of the elongate body 12 is in proximity to the dilated region of the cervix 54. Proper positioning can be aided by feedback provided by the distal pressure sensor 46 when contacting the head 56 of the baby, as well as monitoring the visual feedback from the camera 45. Upon proper positioning, the array of movable elements 24 may be extended to contact the tissue of the cervix 54, for example, by actuating the inflation source 22 to inflate the expandable element 38. As the expandable element 38 is inflated and subsequently expands, the array of movable elements 24 located around the periphery of the expandable element 38 will move outward in a radial direction, while lengths of the plurality of wires 36 will be drawn further into the respective plurality of wire lumens 48. As the array of movable elements 24 is coupled to the plurality of wires 36, which are further coupled to the tension ring 50, the expandable element 38 will expand outward uniformly from the elongate body 12.

The inflation source 22 may continue to inflate the expandable element 38 until the movable elements 24 of the medical device 10 come into contact with the dilated cervix 54. Such contact can be indicated and monitored through information provided by the pressure sensors 32 coupled to the movable elements 24. Furthermore, the control element 20, which is in communication with the sensors, may include an algorithm or computational ability to determine if the pressure sensor feedback indicates a substantially uniform circular state. That is to say, that the pressure measurements from each of the pressure sensors 32 disposed about the movable elements 24 are approximately the same. When the desired inflation level has been attained as indicated by pressure sensor measurements, the inflation source 22 may be deactivated, or, alternatively, the exhaust valve 52 may be triggered to prevent additional fluid from entering the expandable element 38. Once appropriately inflated, the measuring mechanism and the dilation indicator 18 can provide the dilation measurement as indicated by the distance the plurality of wires 36, and thus the tension ring 50, traveled in reaching the expanded state. As previously stated, the dilation indicator 18 can directly correlate the distance traveled by the wires 36, and thus, the measured expansion of the movable elements 24, to an accurate and precise dilation measurement.

Upon completion of the desired measurement, the movable elements 24 are retracted towards the elongate body 12, i.e., by deflating the expandable element 38 by opening the exhaust valve 52, upon which the movable elements 24 will retract to a closed position for the removal of the medical device 10 from the patient. Both the tension ring 50 and the plurality of wires 36 may be biased towards a closed, retracted position, such that when the expandable element 3 8 is not under positive inflation pressure, the medical device 10 retains a closed, retracted state. Furthermore, as described above, the medical device 10 may include an outer sheath 42 which, if used, may be removed and replaced for subsequent uses of the medical device 10, thereby providing a re-usable device while maintaining the sterility of the medical environment.

In an alternative use of the medical device 10 the distal portion of the medical device 10 may be positioned within the cervical region of a patient and be employed to force a safe and uniform dilation where such dilation has not occurred. The medical device 10 could be positioned in the undilated cervix and provide a controllable expansion with a relatively constant pressure provided by the expansion of the expandable element 38. Subsequently, through the monitoring of sensor feedback, the inflation pressure could be appropriately adjusted in order to achieve the desired dilation of the cervical tissue.

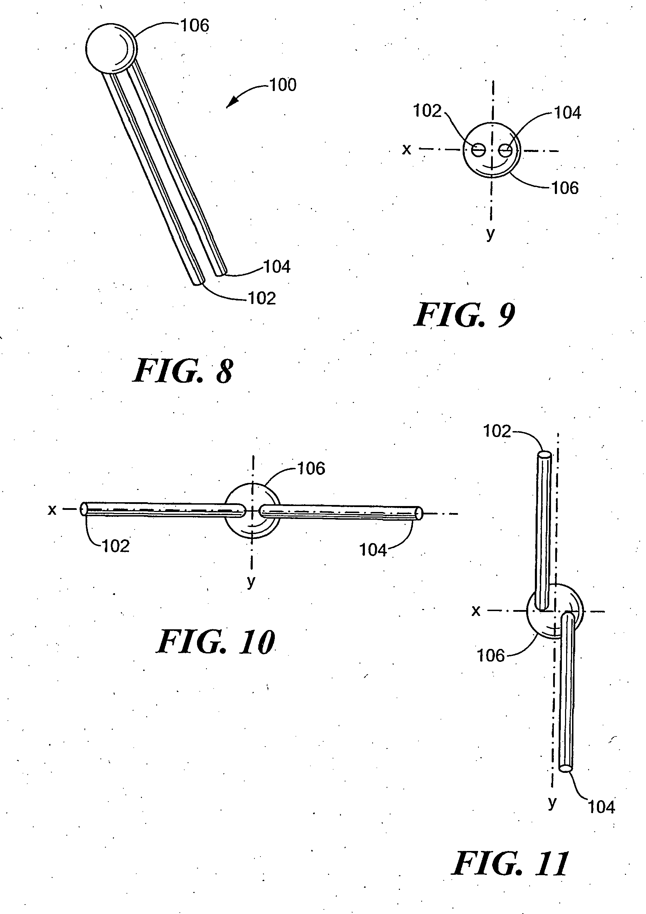

Now referring to FIGS. 8-11, in an embodiment of the present invention, a cervical dilation measurement device 100 is provided to aid in the manual, two-finger approach of measuring cervical dilation. The measurement device 100 includes a first extension element 102, a second extension element 104, and a base element 106. The first and second extension elements 102,104 may be rotatably and pivotably coupled to the base element 106, as to freely move about the housing in at least two planes of motion. The base element 106 may include a dilation indication mechanism to measure the distance between and/or the relative movement of the two extension elements. The dilation indication mechanism may include one or more sensors coupled to or otherwise in communication with the first and second extension elements 102,104. Sensors suitable for monitoring the movement of the first and second extension elements 102, 104 may include sensors mechanically coupled to the extension elements capable of measuring their displacement or movement directly, including but not limited to torque or strain gauges, or may alternatively include sensors positioned in the tips of the first and second extension elements that can monitor distance between the two tips via radiofrequency, optical energy, or the like. A third sensor may be incorporated, in the base element 106 for example, to provide increased accuracy and precision through triangulation methods. The measurement device 100 also includes the control element 20, as previously described and illustrated in FIG. 1, in communication with the base element 106 and one or more sensors for displaying and monitoring information provided by the sensors.

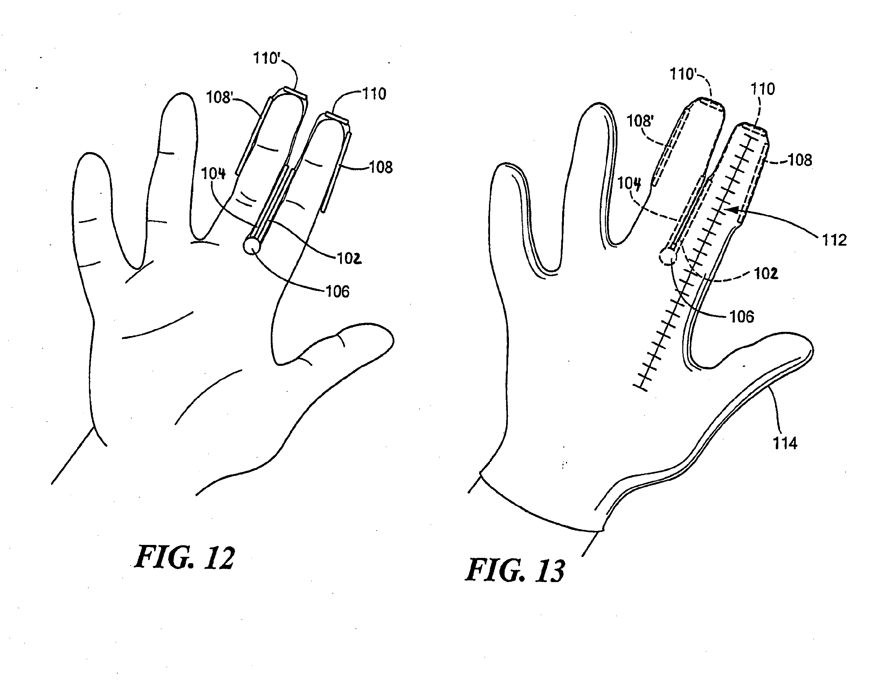

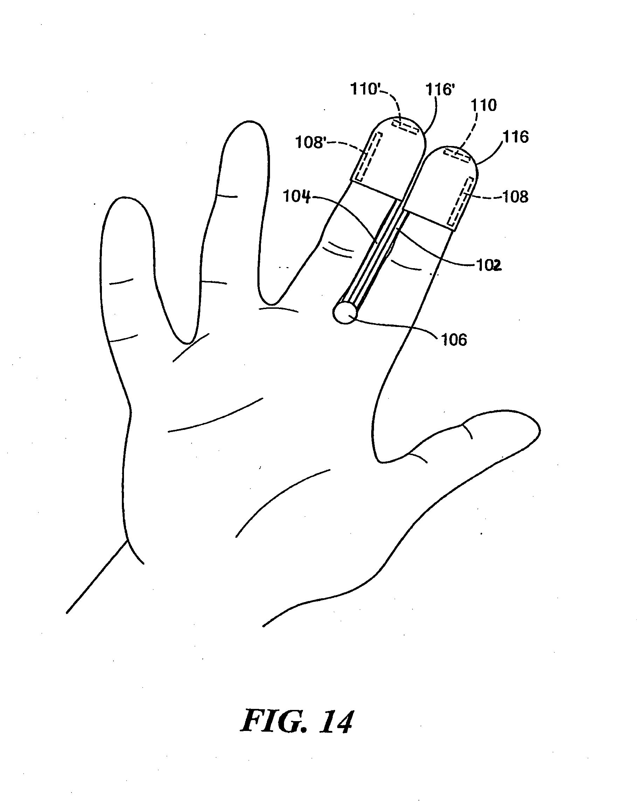

Now referring to FIGS. 12-14, the measurement device 100 of the present invention may also include one or more lateral sensors 108,108' positionable about the sides of the first and second fingers used in the manual cervical dilation measurement technique. The lateral sensors 108,108' may provide pressure feedback information when in contact with the cervix that may assist a physician in making a measurement while avoiding or minimizing cervical distension. As such, the reduced likelihood of cervical distension increases the ability to provide an accurate and precise dilation measurement. The lateral sensors 108, 108' may include one or more thin film pressure sensors, as known in the art, to minimize the increase in width or thickness of the device, thereby providing ease of use and reducing discomfort of the patient, and may further be placed in communication with the control element 20.

The measurement device 100 of the present invention may also include one or more finger-tip pressure sensors 110,110' positionable about the tips of the first and second fingers used in the manual cervical dilation measurement technique. The finger-tip pressure sensors 110, 110' may indicate pressure feedback information via the control element 20 upon contact with the head of the baby. In addition to providing feedback information to prevent excess pressure on the head of the baby, upon recognition that the finger tips are indeed contacting the head of the baby, a marker or other measurement indicator may be used to gauge the position and descent of the baby, as described below.

Historically, practitioners have used the ischial spine as the index point (0 station) for a determination of fetal descent, and assigned an arbitrary number in centimeters above and below the ischial spine. More specifically, "station" refers to the level of the presenting fetal part in the birth canal as described in relationship to the ischial spines, which are halfway between the pelvic inlet and the pelvic outlet. When the lowermost portion of the fetal presenting part is at the level of the ischial spine, it is designated as being at zero (0) station. In the past, the long axis of the birth canal has been arbitrarily divided into segments for a determination of the position of the baby. Thus, as the presenting fetal part descends from the inlet toward the pelvic outlet, the typical designation is -5, -4, -3, -2, -1, 0 station, +1, +2, +3, +4, +5. Using this method, the degree of accuracy (in centimeters) is difficult to achieve clinically. In practice; physicians may generally make an educated guess about the station of the presenting part of the baby, since after the "0" point (0 station), the baby's head covers the ischial spine point and eliminates the ability to measure and reproduce distance caudal to this point. Contrary to the typical method employed, where accuracy and precision may be difficult to maintain, the feedback from the finger-tip sensors may provide an indication of contact with the head of the baby. Upon such indication, a marking or other descent indicator 112 on the portion of the hand of the physician external to the genitalia may be used to provide an accurate and precise measurement of the location and descent of the baby. Measurements over the course of labor indicate rates of progression which are practical, relatively easier to standardize and explainable to the patient or other practitioners. This approach of measurement is termed "Advancement".

In an exemplary use, the measurement device 100 is coupled to the hand of a physician, with the first extension element 102 being paired to a first finger, the second extension element 104 being paired to a second finger, and the base element 106 being positioned in between the first and second fingers. Moreover, where the lateral sensors 108,108' or finger-tip sensors 110, 110' are included, the sensors will be positioned about the sides and tips of the fingers, respectively, as described above. The coupling may be achieved through the integration of the measurement device 100 with a glove 114, or through direct adhesion of the various components to the fingers themselves. Additionally, the cervical dilation measurement device 100 may include two cap elements 116,116' positionable about the finger tips, with the first and second extension elements 102,104 extending from the cap elements 116,116' and towards the base element 106, and with the lateral and finger-tip sensors coupled to the cap elements in the appropriate positions. Any wires or other communicative elements connecting the sensors to the control element 20 may be routed through the glove or positioned down the back of the hand as needed to provide connectivity while preventing interference with the use of the device. Alternatively, the various sensors may communicate with the control element 20 wirelessly as known in the art.

Subsequently, the physician may position the first and second fingers and the cervical dilation measurement device 100 in proximity to the cervix. Upon reaching the desired location, the two fingers can be spread either into a "V" shape or an "L" shape, and the relative movement of the first and second extension elements 102,104 may be measured by the one or more sensors in the base element 106, with the lateral sensors 108,108' preventing cervical distension as previously described. As a result, the physician will not be required to make a subjective observation as to the actual cervical dilation, as the actual width between the spread fingers can be accurately assessed by the cervical dilation measurement device 100 and provided to the physician through the control element 20. In addition, upon contacting the head of the baby with the finger-tip sensors, the descent indicator 112 may be referenced to determine the location of the baby.

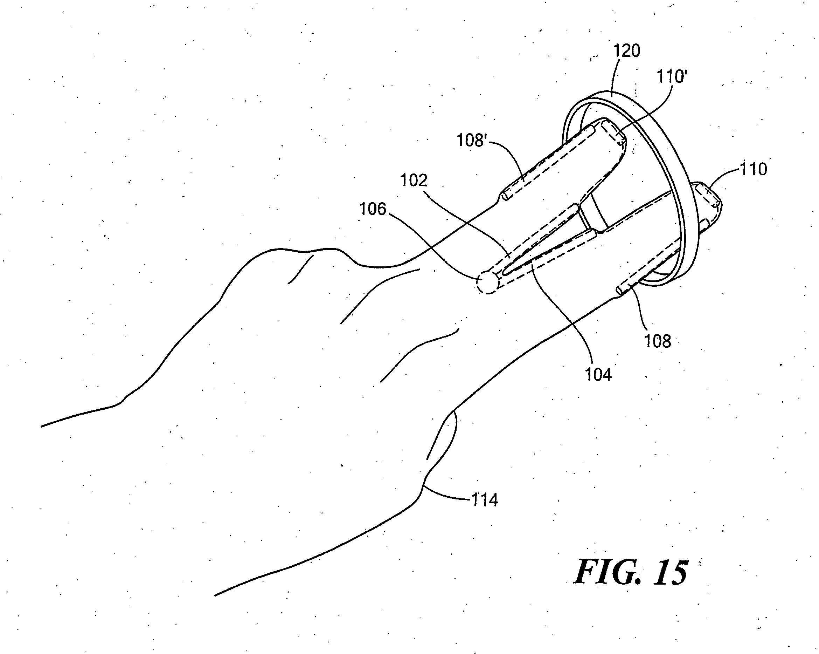

While the method of measurement as described above may provide an accurate and precise measurement of cervical dilation, it is realized that different physicians may have variations in both finger length and thickness which may affect the accuracy of the measured dilation. Now referring to FIG. 15, the present invention may include a calibration element 120 for use with the measurement device 100 to compensate for the variations in the finger dimensions of a physician. The calibration element 120 may include an object of known dimensions, thereby providing a reference value from which the measurement device 100 may be calibrated. For example, the measurement device 100 may be coupled or otherwise positioned about the hand of a physician or operator, with the first extension element 102 being paired to a first finger, the second extension element 104 being paired to a second finger, and the base element 106 being positioned in between the two fingers. Subsequently, the first and second fingers may be extended such that an outer portion of the first and second fingers contact a portion of the calibration element 120, providing a "simulated" distance measurement. Upon contacting the calibration element 120, the first and second fingers will be separated by a known distance, and the relative movement of the first and second extension elements 102,104 about the base element 106 can be appropriately modified to reflect an accurate and precise measurement. Such modification may include, for example, an algorithm or other computational calculation taking into account the known, fixed dimensions of the calibration element 120, the known length of the first and second extension elements 102,104, as well as the angle formed between them at the intersection with the base element 106. The suggested calibration procedure may be performed a single time for each operator who may thereafter use the measurement device 100, and such values and calibration modifications may be stored in the control element 20 for ease of subsequent use without the need to re-calibrate the device. Alternatively, the suggested calibration procedure may be performed prior to each dilation measurement to ensure accuracy and precision.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope of the invention, which is limited only by the following claims.

## Claims

1. Medical device for measuring cervical dilation during delivery of a baby, the medical device being disposed within a glove (114) and positionable about the fingers of a human hand,
wherein the fingers of a human hand have a top nail bed portion at the distal end of the fingers, a palm portion at the proximal end of the fingers, a pad portion on the same face of the hand as the palm portion, a tip portion, and side portions, the medical device comprising:
a base (106) positionable between an index finger and a middle finger at the proximal end of the fingers;
a first extension element (102) movably coupled to the base (106) and positioned on the side portion of the index finger that faces the middle finger, wherein the first extension element (102) is movable about the base (106) in at least two planes of motion;
a second extension element (104) movably coupled to the base (106) and positioned on the side portion of the middle finger that faces the index finger, wherein the second extension element (104) is movable about the base (106) in at least two planes of motion; and
a control element (20) in communication with the base (106), to provide a measurement of a distance between the first and second extension element (102, 104).

2. Medical device according to claim 1, **characterized by** a first finger-tip pressure sensor (110) positionable on the tip portion of the index finger, wherein the first finger-tip pressure sensor (110) is in communication with the control element (20).

3. Medical device according to claim 2, **characterized by** a second finger-tip pressure sensor (110') positionable on the tip portion of the middle finger, wherein the second finger-tip pressure sensor (110') is in communication with the control element (20).

4. Medical device according to claim 1, **characterized by** a first pressure sensor (108) in communication with the control element (20), wherein the first pressure sensor (108) is positionable along a side portion of the index finger that faces away from the middle finger.

5. Medical device according to claim 4, **characterized by** a second pressure sensor (108') in communication with the control element (20), wherein the second lateral pressure sensor (108') is positionable along a side portion of the middle finger that faces away from the index finger.

6. Medical device according to claim 1, **characterized in that** the base (106) and the first and second extension element (102, 104) are coupled to an interior portion of the glove (114).

7. Medical device according to claim 6, **characterized in that** the glove (114) includes a descent indicator (112).

8. Medical device according to claim 1, **characterized by** first and second pressure sensors (108, 108') coupled to an interior portion of the glove (114) and positionable along side portions of the index and middle fingers, respectively, wherein the first and second pressure sensors (108, 108') are in communication with the control element (20).

9. Medical device according to claim 1, **characterized by** first and second finger-tip pressure sensors (110, 110') coupled to the glove (114) and positionable on tip portions of the index and middle fingers, respectively, wherein the first and second finger-tip pressure sensors (110, 110') are in communication with the control element (20).

10. Medical device according to claim 1, **characterized by** a first cap element (116) coupled to the first extension element (102) and engageable with the tip portion of the index finger, and a second cap element (116') coupled to the second extension element (104) and engageable with the tip portion of the middle finger.

11. Medical device according to claim 10, **characterized by** a first pressure sensor (108) coupled to the first cap element (116), wherein the first pressure sensor (108) is positionable along a side portion of the index finger, and a second pressure sensor (108') coupled to the second cap element (116'), wherein the second pressure sensor (108') is positionable along a side portion of the middle finger.

12. Medical device according to claim 10, **characterized by** a first finger tip pressure sensor (110) positionable on the tip portion of the index finger and coupled to the first cap element (116), and a second finger-tip pressure sensor (110') positionable on the tip portion of the middle finger and coupled to the second cap element (116').

13. Medical device according to claim 1, **characterized by** a first pressure sensor (108) positionable along a side portion of the index finger and in communication with the control element (20); a second pressure sensor (108') positionable along a side portion of the middle finger and in communication with the control element (20); a third pressure sensor (110) positionable on the tip portion of the index finger and in communication with the control element (20); and a fourth pressure sensor (110') positionable on the tip portion of the middle finger and in communication with the control element (20).

14. Medical device according to claim 13, **characterized in that** the base (106), first extension element (102), second extension element (104), first pressure sensor (108), second pressure sensor (108'), third pressure sensor (110), and fourth pressure sensor (110') are coupled to the glove (114).

15. Medical device according to claim 14, **characterized in that** the glove (114) includes a descent indicator (112).

16. Medical device according to claim 13, **characterized by** a first cap element (116) coupled to the first extension element (102) and engageable with the tip portion of the index finger, and a second cap element (116') coupled to the second extension element (104) and engageable with the tip portion of the middle finger.

## Patentansprüche

1. Medizinisches Gerät zum Messen der zervikalen Dilatation während der Geburt eines Kindes, wobei das medizinische Gerät in einem Handschuh (114) angeordnet und auf den Fingern einer menschlichen Hand positionierbar ist, und die Finger einer menschlichen Hand an dem entfernt liegenden Ende der Finger einen oberen Nagelbettteil aufweisen, an dem nahe gelegenen Ende der Finger einen Handflächenteil, des weiteren einen Ballenteil auf derselben Fläche der Hand wie der Handflächenteil, einen spitzen Teil sowie Seitenteile, und wobei das medizinische Gerät des weiteren folgendes umfaßt: einen Grundkörper (106), der zwischen einem Zeigefinger und einem Mittelfinger an dem nahe gelegenen Ende der Finger angeordnet ist; ein erste Verlängerungselement (102), das mit dem Grundkörper (106) beweglich gekoppelt ist und sich auf dem Seitenteil des Zeigefingers befindet, der dem Mittelfinger zugewendet ist, wobei das erste Verlängerungselement (102) um den Grundkörper (106) in wenigstens zwei Bewegungsebenen beweglich ist; ferner umfassend ein zweites Verlängerungselement (104), das mit dem Grundkörper (106) beweglich gekoppelt ist und sich auf dem Seitenteil des Mittelfingers befindet, der dem Zeigefinger zugewendet ist, wobei das zweite Verlängerungselement (104) um den Grundkörper (106) in wenigstens zwei Bewegungsebenen beweglich ist; schließlich umfassend ein Steuerelement (20), das mit dem Grundkörper (106) in Verbindung steht, um zwischen dem ersten Verlängerungselement (102) und dem zweiten Verlängerungselement (104) eine Distanzmessung zu ermöglichen.

2. Medizinisches Gerät nach Anspruch 1, **gekennzeichnet durch** einen ersten Fingerspitzen-Drucksensor (110), der auf dem Spitzenteil des Zeigefingers angeordnet werden kann, wobei der erste Fingerspitzen-Drucksensor (110) mit dem Steuerelement (20) als solchem in Verbindung steht.

3. Medizinisches Gerät nach Anspruch 2, **gekennzeichnet durch** einen zweiten Fingerspitzen-Drucksensor (110'), der auf dem Spitzenteil des Mittelfingers angeordnet werden kann, wobei der zweite Fingerspitzen-Drucksensor (110') mit dem Steuerelement (20) in Verbindung steht.

4. Medizinisches Gerät nach Anspruch 1, **gekennzeichnet durch** einen ersten Drucksensor (108), der mit dem Steuerelement (20) verbunden ist, wobei der erste Drucksensor (108) längs eines Seitenteils des Zeigefingers angeordnet werden kann, der von dem Mittelfinger wegweist.

5. Medizinisches Gerät nach Anspruch 4, **gekennzeichnet durch** einen zweiten Drucksensor (108'), der mit dem Steuerelement (20) in Verbindung steht, wobei der zweite seitliche Drucksensor (108') längs eines Seitenteils des Mittelfingers angeordnet werden kann, der von dem Zeigefinger wegweist.

6. Medizinisches Gerät nach Anspruch 1, **dadurch gekennzeichnet, daß** der Grundkörper (106) und das erste sowie das zweite Verlängerungselement (102, 104) mit einem inneren Teil des Handschuhs (114) gekoppelt sind.

7. Medizinisches Gerät nach Anspruch 6, **dadurch gekennzeichnet, daß** der Handschuh (114) einen Geburtsindikator (112) aufweist.

8. Medizinisches Gerät nach Anspruch 1, **gekennzeichnet durch** erste und zweite Drucksensoren (108, 108'), die mit einem inneren Teil des Handschuhs (114) verbunden sind und entlang der Seitenteile des Zeige- bzw. Mittelfingers angeordnet werden können, wobei die ersten und zweiten Drucksensoren (108, 108') mit dem Steuerelement (20) verbunden sind.

9. Medizinisches Gerät nach Anspruch 1, **gekennzeichnet durch** erste und zweite Fingerspitzen-Drucksensoren (110, 110'), die mit dem Handschuh (114) gekoppelt sind und auf den Spitzenteilen des Zeigefingers bzw. Mittelfingers angeordnet werden können, wobei die ersten und zweiten Fingerspitzen-Drucksensoren (110, 110') mit dem Steuerelement (20) in Verbindung stehen.

10. Medizinisches Gerät nach Anspruch 1, **gekennzeichnet durch** ein erstes Kuppenelement (116), das mit dem ersten Verlängerungselement (102) gekoppelt ist und mit dem Spitzenteil des Zeigefingers in Eingriff steht, und ein zweites Kuppenelement (116'), das mit dem zweiten Verlängerungselement (104) gekoppelt ist und mit dem Spitzenteil des Mittelfingers in Berührung steht.

11. Medizinisches Gerät nach Anspruch 10, **gekennzeichnet durch** einen ersten Drucksensor (108), der mit dem ersten Kappenelement (116) gekoppelt ist, wobei der erste Drucksensor (108) entlang eines Seitenteils des Zeigefingers anordbar ist, und einen zweiten Drucksensor (108), der mit dem zweiten Kuppenelement (116') gekoppelt ist, wobei der zweite Drucksensor (108') entlang eines Seitenteils des Mittelfingers anordbar ist.

12. Medizinisches Gerät nach Anspruch 10, **gekennzeichnet durch** einen ersten Fingerspitzen-Drucksensor (110), der auf dem Spitzenteil des Zeigefingers anordbar ist und mit dem ersten Kuppenelement (116) gekoppelt ist, und einen zweiten Fingerspitzen-Drucksensor (110'), der auf dem Spitzenteil des Mittelfingers anordbar ist und mit dem zweiten Kuppenelement (116') verbunden werden kann.

13. Medizinisches Gerät nach Anspruch 1, **gekennzeichnet durch** einen ersten Drucksensor (108), der entlang eines Seitenteils des Zeigefingers anordbar ist und mit dem Steuerelement (20) verbunden ist; einen zweiten Drucksensor (108'), der entlang eines Seitenteils des Mittelfingers anordbar ist und mit dem Steuerelement (20) verbunden werden kann; einen dritten Drucksensor (110), der auf dem Spitzenteil des Zeigefingers anordbar ist und mit dem Steuerelement (20) verbunden ist; und einen vierten Drucksensor (110'), der auf dem Spitzenteil des Mittelfingers anordbar ist und mit dem Steuerelement (20) in Verbindung steht.

14. Medizinisches Gerät nach Anspruch 13, **dadurch gekennzeichnet, daß** der Grundkörper (106), das erste Verlängerungselement (102), das zweite Verlängerungselement (104), der erste Drucksensor (108), der zweite Drucksensor (108'), der dritte Drucksensor (110) und der vierte Drucksensor (110') mit dem Handschuh (114) gekoppelt sind.

15. Medizinisches Gerät nach Anspruch 14, **dadurch gekennzeichnet, daß** der Handschuh (114) einen Geburtsindikator (112) aufweist.

16. Medizinisches Gerät nach Anspruch 13, **gekennzeichnet durch** ein erste Kuppenelement (116), das mit der ersten Verlängerung (102) gekoppelt ist und mit dem Spitzenteil des Zeigefingers in Berührung steht, und ein zweites Kuppenelement (116'), das mit dem zweiten Verlängerungselement (104) gekoppelt ist und mit dem Spitzenteil des Mittelfingers in Berührung bringbar ist.

## Revendications

1. Dispositif médical pour mesurer la dilatation du col de l'utérus pendant l'accouchement d'un bébé, le dispositif médical étant disposé à l'intérieur d'un gant (114) est susceptible d'être positionné autour des doigts d'une main humaine,
dans lequel les doigts d'une main humaine comprennent une portion formant base d'ongle à l'extrémité distale des doigts, une portion de paume à l'extrémité proximale des doigts, une portion de coussinet sur la même face de la main que la portion de paume, une portion de bout de doigt, et des portions latérales, le dispositif médical comprenant :
une base (106) susceptible d'être positionnée entre un index et un majeur à l'extrémité proximale des doigts ;
un premier élément d'extension (102) couplé de façon mobile à la base (106) et positionné sur la portion latérale de l'index qui fait face vers le majeur, dans lequel le premier élément d'extension (102) est déplaçable autour de la base (106) dans au moins deux plans de mouvement ;
un second élément d'extension (104) couplé de façon mobile à la base (106) et positionné sur la portion latérale du majeur qui fait face vers l'index, dans lequel le second élément d'extension (104) est déplaçable autour de la base (106) dans au moins deux plans de mouvement ; et
un élément de commande (20) en communication avec la base (106) pour fournir une mesure d'une distance entre le premier et le second élément d'extension (102, 104).

2. Dispositif médical selon la revendication 1, **caractérisé par** un premier capteur de pression de bout de doigt (110) susceptible d'être positionné sur la portion de bout de doigt de l'index, dans lequel le premier capteur de pression de bout de doigt (110) est en communication avec l'élément de commande (20).

3. Dispositif médical selon la revendication 2, **caractérisé par** un second capteur de pression de bout de doigt (110') susceptible d'être positionné sur la portion de bout de doigt du majeur, dans lequel le second capteur de pression de bout de doigt (110') est en communication avec l'élément de commande (20).

4. Dispositif médical selon la revendication 1, **caractérisé par** un premier capteur de pression (108) en communication avec l'élément de commande (20), dans lequel le premier capteur de pression (108) est susceptible d'être positionné le long d'une portion latérale de l'index qui est détournée du majeur.

5. Dispositif médical selon la revendication 4, **caractérisé par** un second capteur de pression (108') en communication avec l'élément de commande (20), dans lequel le second capteur de pression latérale (108') est susceptible d'être positionné le long d'une portion latérale du majeur qui est détournée de l'index.

6. Dispositif médical selon la revendication 1, **caractérisé en ce que** la base (106) et le premier et le second élément d'extension (102, 104) sont couplés à une portion intérieure du gant (114).

7. Dispositif médical selon la revendication 6, **caractérisé en ce que** le gant (114) inclut un indicateur de descente (112).

8. Dispositif médical selon la revendication 1, **caractérisé par** un premier et un second capteur de pression (108, 108') couplés à une portion intérieure du gant (114) est susceptible d'être positionnés le long de portion latérale de l'index et du majeur, respectivement, dans lequel le premier et le second capteur de pression (108, 108') sont en communication avec l'élément de commande (20).

9. Dispositif médical selon la revendication 1, **caractérisé par** un premier et un second capteur de pression de bout de doigt (110, 100') couplés au gant (114) est susceptibles d'être positionnés sur les portions de bout de doigt de l'index et du majeur, respectivement, dans lequel le premier et le second capteur de pression de bout de doigt (110, 110') sont en communication avec l'élément de commande (20).

10. Dispositif médical selon la revendication 1, **caractérisé par** un premier élément de capuchon (116) couplé au premier élément d'extension (102) est susceptible d'être engagé avec la portion de bout de doigt de l'index, et par un second élément de capuchon (116') couplé au second élément d'extension (104) est susceptible d'être engagé avec la portion de bout de doigt du majeur.

11. Dispositif médical selon la revendication 10, **caractérisé par** un premier capteur de pression (108) couplé au premier élément de capuchon (116), dans lequel le premier capteur de pression (108) est susceptible d'être positionné le long d'une portion latérale de l'index, et par un second capteur de pression (108') couplé au second élément de capuchon (116'), dans lequel le second capteur de pression (108') est susceptible d'être positionné le long d'une portion latérale du majeur.

12. Dispositif médical selon la revendication 10, **caractérisé par** un premier capteur de pression de bout de doigt (110) susceptible d'être positionné sur la portion de bout de doigt de l'index et couplé au premier élément de capuchon (116), et par un second capteur de pression de bout de doigt (110') susceptible d'être positionné sur la portion de bout de doigt du majeur et couplé au second élément de capuchon (116').

13. Dispositif médical selon la revendication 1, **caractérisé par** un premier capteur de pression (108) susceptible d'être positionné le long d'une portion latérale de l'index et en communication avec l'élément de commande (20) ; par un second capteur de pression (108') susceptible d'être positionné le long d'une portion latérale du majeur et en communication avec l'élément de commande (20) ; par un troisième capteur de pression (110) susceptible d'être positionné sur la portion de bout de doigt de l'index et en communication avec l'élément de commande (20) ; et par un quatrième capteur de pression (110') susceptible d'être positionné sur la portion de bout de doigt du majeur et en communication avec l'élément de commande (20).

14. Dispositif médical selon la revendication 13, **caractérisé en ce que** la base (106), le premier élément d'extension (102), le second élément d'extension (104), le premier capteur de pression (108), le second capteur de pression (108'), le troisième capteur de pression (110), et le quatrième capteur de pression (110') sont couplés au gant (114).

15. Dispositif médical selon la revendication 14, **caractérisé en ce que** le gant (114) inclut un indicateur de descente (112).

16. Dispositif médical selon la revendication 13, **caractérisé par** un premier élément de capuchon (116) couplé au premier élément d'extension (102) et susceptible d'être engagé avec la portion de bout de doigt de l'index, et par un second élément de capuchon (116') couplé au second élément d'extension (104) et susceptible d'être engagé avec la portion de bout de doigt du majeur.
